# EUROPEAN PATENT SPECIFICATION

(11) **EP 3 581 172 B1**
(45) Date of publication and mention of the grant of the patent: **20.12.2023**
(21) Application number: 19179719.0
(22) Date of filing: 12.06.2019
(51) Int. Cl.: A61K 36/9066, A61K 31/12, A61K 33/04, A61K 9/20, A61K 9/00

(54) **A PHARMACEUTICAL COMPOSITION WITH ENHANCED BIOAVAILABILITY CONTAINING A MEDICAL HERBAL EXTRACT**
PHARMAZEUTISCHE ZUSAMMENSETZUNG MIT VERBESSERTER BIOVERFÜGBARKEIT MIT EINEM MEDIZINISCHEN KRÄUTEREXTRAKT
COMPOSITION PHARMACEUTIQUE À BIODISPONIBILITÉ ACCRUE CONTENANT UN EXTRAIT VÉGÉTAL MÉDICAL

(30) Priority: 14.06.2018 TR 201808526
(43) Date of publication of application: 18.12.2019
(73) Proprietor: TFLL Pharmaceutical Food Supplements and Cosmetic Products - Industry Foreign Trade, 34746 Istanbul (TR)
(72) Inventor: Simsek, Serhan, 34882 Istanbul (TR); Simsek, Semra Aktas, 34746 Istanbul (TR)
(74) Representative: Arnold & Siedsma

(56) References cited:
- WO-A1-2009/144220
- US-A1- 2006 198 906
- US-A1- 2013 017 182
- US-A1- 2017 202 238
- US-A1- 2018 007 945
- US-A1- 2018 169 071
- Anonymous: "Men's Multi Dietary Supplement", , 30 September 2015 (2015-09-30), XP55325921, Retrieved from the Internet: URL:http://www.gnpd.com/sinatra/recordpage /3475423/from_search/xWN9afrKjU/?page=1 [retrieved on 2016-12-05]
- DATABASE WPI Week 201479 Thomson Scientific, London, GB; AN 2014-V44327 XP002794740, & CN 104 013 601 A (UNIV CHONGQING MEDICAL) 3 September 2014 (2014-09-03)

## Description

### Technical Field

The present invention relates to a pharmaceutical composition intended for human use as a food supplement with high absorption and bioavailability, consisting of the combinations of natural herbal substances and minerals.

### Background Art

### Curcumin

The turmeric plant known as curcuma (curcuma longa) has been used for centuries as a spice in Indian cuisine and in traditional medical methods in the Chinese medicine.

Turmeric (curcuma longa), also known as 'curcuma domestica', is a perennial herbaceous plant of the ginger family (Zingiberaceae). Although it has more than 300 active components, a substance obtained from its root that has the feature of being a yellow or orange pigment is the main biologically active component constituting the basis for the medicinal properties of this plant. Called as curcumin about 200 years ago, this substance called curcumin, is also the main component of curry powder commonly used in Asian cuisine. It is also used as a food colorant with the code E100.

Curcumin is the most important of the major bioactive components derived from the rhizomes (Rhizoma Curcumae) of the Turmeric (Curcuma longa) plant, which is a yellow-flowered, large-leaved, perennial herbaceous plant of the ginger family (Zingiberaceae). Curcumin is employed in traditional medicine for the treatment of various diseases such as indigestion, urinary tract infections, liver diseases and rheumatoid arthritis.

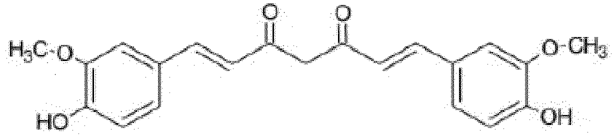

### Curcumin

### [(1E,6E)-1,7-bis (4-hydroxy-3-methoxyphenyl)-1,6-heptadiene-3,5-dione]

Curcuminoids constitute the active ingredient group found in the roots of turmeric. And the most important and potent member of this group is curcumin. Curcumin is oil-soluble and has strong anti-inflammatory and antioxidant effects. Osteoarthritis and rheumatoid arthritis are the two major disorders in which curcumin provides a strong anti-inflammatory effect. It alleviates joint pain, inflammation and joint sensitivity, and prevents joint damage. Curcumin has an efficacy similar to some NSAIDs, but with a lower side-effect profile.

Thanks to its broad spectrum effects, curcumin is also preferred for the treatment of inflammation and autoimmune diseases in the body. Among relevant disorders addressed in recent studies are rheumatoid arthritis, IBS, cardiovascular diseases, depression, Alzheimer's disease and cancer.

Studies highlight that curcumin has positive and promising results in the treatment of cancer. Curcumin has increasingly been the centre of interest in cancer research and treatment for its ability to inhibit carcinogenesis, chemopreventive nature and ability to reduce the metastatic rate.

Chronic inflammation is the major factor in the development of major degenerative diseases in modern societies such as dementia, diabetes and obesity. Research is particularly being focussed on anti-inflammatory nutrition to prevent these diseases. The anti-inflammatory role and potential effects of phytonutrients in the prevention of modern life diseases is increasingly attracting attention.

Many laboratory studies have revealed some anti-cancer properties of curcumin both for treatment and prophylaxis. In the literature, there are many laboratory studies investigating the antiproliferative effects of curcumin in various tumour cells. In one of them; curcumin inhibited cell proliferation in the cell lines of uterine cancer. In another study on mice; curcumin significantly inhibited proliferation in prostate cancer cells.

Again in a study on mice; after feeding a portion of the mice with curcumin, mice were injected with a carcinogenic substance that can cause cancer in the esophagus, and as a result, it was found that cell proliferation markers were significantly lower in curcumin-fed mice.

Studies have shown that, as an anticancer agent, it suppresses tumour formation in many types of cancer. Derived from turmeric, curcumin has been used for the treatment of various inflammatory diseases for centuries. It is believed that turmeric acts by intervening in the cell cycle (cyclin D1 and cyclin E), apoptosis (activation of caspases and reduction of receptors in antiapoptotic genes), reproduction (HER-2, EGFR, AP-1), survival (PI3K/AKT pathway), proliferation (MMP-9 and adhesion molecules), formation of new vasculature (VEGF), metastasis (CXCR-4), inflammation (NF-KB, TNF, IL-6, IL-1, COX- 2, AND 5-LOX) and their multiple cell signalling pathways. Although traditional herbal remedies are considered safe, their active principles and how they mediate against cancer are not exactly known. Thanks to their free radical scavenging properties, phenolic compounds exhibit antioxidant and anticancer activity.

Curcumin has excellent antioxidant activity thanks to its phenolic and enolic functional groups. Studies have verified that oxygenated aromatic rings of curcumin and analogs thereof have cytostatic activity.

Curcumin's anti-neoplastic activity, low molecular weight and no toxicity renders it as an ideal precursor molecule in the development of potential chemotherapeutic drugs.

### 1. Anticancer Potential of Curcumin

### 1.1. Breast Cancer

Studies report that the antiproliferative effect of curcumin in human breast cancer cell is associated with the inhibition of ornithine decarboxylase activity by curcumin. Some mechanisms of action of curcumin in breast cancer cells have been described. For example; it has been found to inhibit aryl hydrocarbon receptor and cytochrome P450 1A1; p185neu glycoprotein with tyrosine kinase activity; Ki-67, PCNA and p53 mRNA expression; and COX-1 and COX-2 enzymes. Curcumin also induces p-53 dependent Bax expression, and inhibits vascular endothelial growth factor (VEGF) and basic fibroblast growth factor (b-FGF).

Human telomerase reverse transcriptase has been shown to completely inhibit telomerase activity. It reduces the expression of matrix metalloproteinase-2 (MMP-2), increases the tissue inhibitor metalloproteinase-1 (TIMP-1) and blocks NF-2B and AP-1 activation. Studies have also shown that curcumin inhibits the LOX pathway and suppresses insulin-like growth factor (IGF-1) [15] in breast cancer cell lines. In early clinical trials, a significant reduction in lesion size, pain, itching and leaks were recorded as positive responses in 71% of the 7 breast cancer patients after the administration of topical curcumin ointment.

### 1.2. Esophageal Cancer

Due to the side effects caused by treatments such as the preferred surgical intervention, such treatments do not yield positive results in terms of survival rate. For example; pneumonia, postoperative pulmonary complications, pharynx inflammation and rheumatoid inflammation are serious side effects [16]. There is need to the change and improvement of treatment strategies. Curcumin was found to inhibit iNOS, JNK, VCAM and NF-κBs in cytokine- stimulating activity in esophageal microvascular endothelial cells isolated from the normal human esophageal tissue. Experiments report that when rats were administrated by diet (500 ppm) an onset dose followed by a maintenance dose of curcumin, esophageal carcinogenesis was inhibited by 27% and 33% respectively.

### 1.3. Gastric Cancer

Current treatment modalities substantially include surgical intervention and chemotherapy, however relapse and metastasis are common. There is need to change the treatment strategies. It has been determined that curcumin has a cytotoxic effect on gastric cancer cells. Curcumin has been found to inhibit growth of gastric cancer cells by synergistic action with 5-fluorouracil (5-FU). In another study, curcumin was associated with the reversal of MDR (multidrug resistance), decrease in P-gp function in human gastric cancer cells and increase in caspase-3 activation.

### 1.4. Intestinal Cancer

Examining the intestinal tissues of curcumin-treated animals, the anti-tumor effect of curcumin was associated with the increase in enterocyte apoptosis and proliferation.

Administering the rats with curcumin by diet in an amount of 0.2% and 0.5% for a period of 15 weeks resulted in a decrease in adenoma by 39% and 40% respectively. In one study, a delay was observed in the growth of adenoma during the expression of COX-2 protein after treatment with curcumin in mouse intestines.

### 1.5. Liver Cancer

Curcumin inhibits cell cycle in liver cancer cells, shows cytotoxic effect and also has antiproliferative and apoptosis stimulating effects. Curcumin has a strongly inhibitory effect on phenol sulfotransferase (SULT1A1) in human liver and extrahepatic tissues. Curcumin inhibits IL-6 production, histone acetyltransferase (HAT) activity, and AP-1 activation. Curcumin is believed to inhibit tumor growth in hepatocellular carcinoma (HCC) by degrading HIF1, the aryl hydrocarbon receptor translocator. The anticarcinogenic effect of curcumin may also be shown by its inhibition of glutathione-associated detoxification enzymes in rat liver. The anti-vascularity effect of curcumin in hepatocarcinoma cells implanted into the liver of mice is shown by its mediating the reduction of both COX and VEGF.

### 1.6. Pancreatic Cancer

It has been found to inhibit the activity of farnesyl protein transferase in MIA PaCa-2 cells in human pancreatic cancer. It has been reported that NF-κB is abundant in human pancreatic tutor tissues and cell pathways, and that curcumin has the ability to suppress NF-κB expression. Likewise, curcumin further reduces a wide range of IL-8 bioactivity, which contributes to tumour growth and the viability of pancreatic cancer cells. In patients, curcumin was reported to reduce the expression of phosphorylated STAT3 in NF-κB, COX-2 and PBMC.

### 1.7. Colorectal Cancer

Curcumin has been found to inhibit proliferation and induce apoptosis in colorectal cell pathways. Some events caused by curcumin include chromatin condensation and DNA lysis, repair of DNA damage in HT-29 cells and HCT-116 colon lymphocytes, as well as GADD153 mRNA and protein expression [31, 5]. Curcumin inhibits neurotensin-mediated activator inhibits protein-1, NF-κB activation, calcium mobilization, PGE-2 and EGFR; and reduces COX-1 and-2, MMP-2 and-9, IL-8 gene induction and migration of colon cancer cells.

Some curcumin derivatives have been found to be effective against colon cancer cells.

For example, compared to curcumin, dimethoxycurcumin (shown below left) is more effective in reducing proliferation and stimulating apoptosis in HCT116 cells.

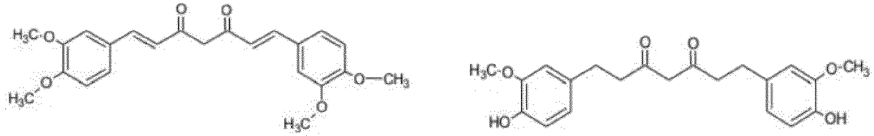

### Dimethoxycurcumin Tetrahydrokurkuminin

An assessment of the effects of tetrahydrokurkuminin (THC) (Shown above right) on colon cancer stimulated by 1,2-dimethylhydrazine (DMH) has revealed that THC has caused a significant decrease in both upper and lower half sections of colon crypts.

Research shows that curcumin and curcumin analogs are effective in many types of cancer. No toxic effect on normal cells, high anti-neoplastic activity and low molecular weight render this molecule an ideal precursor in the development of potential chemotherapeutic compounds. However, given its current low bioavailability, the potential use of curcumin will progressively enhance with the development of new dosage forms that offer higher bioavailability.

Curcumin is characterized as a lipophilic (fat-soluble) yellow substance. Its water solubility is almost negligible.

Curcumin-, dihydrocurcumin-, and tetrahydrocurcuminglucuronides and tetrahydrocurcumin are the main metabolites *in vivo.* Blood concentrations are very low after oral administration because of its rapid metabolism in human liver and small intestinal wall. Even with an oral dose of 10-12 g, it can be obtained in the nanomolar range and below 160 nm/ml in blood.

Because of its many therapeutic potentials mentioned above, primarily cancer, the investigators have been studying for long on enhancing the absorption and phytochemical retention of this substance. These studies are primarily listed below:
- Slowing down the metabolism of curcumin
- Improving oral liquid systems
- Micronization of curcumin
- Combination with piperine

None of these options help to obtain curcumin with the desired high bioavailability.

In order to slow down the curcumin metabolism, an adjuvant should strictly be co-administered with the curcumin to be administered orally, however its resulting clinical effects are not known.

In oral liquid systems, oral intake of too much surfactant is needed, posing the risk of loss of positive clinical effects expected from curcumin or encountering other problems due to potential side effects.

Further, micronization does not increase solubility and does not resolve oral absorption and absorption problems.

On the other hand, combination with piperine is a very common method, however the modifying effect of many active ingredients of piperine on nutritional supplementation and drug metabolism has a limiting effect on patients who need to use other drugs in case of co-administration.

Furthermore, there are also oral dispersible dosage forms in the prior art. One of them is commercially marketed by MCE PHARMA under the name "oral dissolution tablet". This product includes the features listed below.
1. Contains submicron curcumin particles.
2. Lecithin and piperine are used in the formulation.
3. Vitamin D3 is used in the formulation.
4. Prepared in the form of an orally-soluble formulation.

As previously mentioned, above characteristics fail to achieve high bioavailability. It has been proven by many scientific publications that curcumin is degraded in stability with the reduction of particle size. The effects of particle size change on stability are still under research. (Microparticles Containing Curcumin Solid Dispersion: Stability, Bioavailability and Anti-Inflammatory Activity ;C.Teixeira, L. M. Mendonça, M. M. Bergamaschi, R. H. C. Queiroz, G. E. P. Souza, L. M. G. Antunes, and L. A. P. Freitas; AAPS 15 PharmSciTech. 2016 Apr; 17(2): 252-261.)

### REFERENCES

1. Anand, P., Sundaram. C., Jhurani. S. Kunnumakkara, A.B.. Aggarwal. B.B. (2008). Curcumin and cancer: An "old-age" disease with an "age-old" solution. Cancer Letters. 267, 133-164.
2. Cooper. T. H.. Clark. J.G.. Guzinski. J.A. (1994). In food phytochemicals for cancer prevention II: ACS Symposium Series: Ho. C.-T., Ed.; American Chemical Society: New York. 547. pp 231-236.
3. Sharma. R. A., Gescher, A.J., Steward, W.P. (2005). Curcumin: the story so far. European Journal of Cancer. 41. 1955-1968.
4. Youssef, D., Nichols. C. E.. Cameron, T.. Cameron. S.. Balzarini. J.. De Clercq, E.. Jha, A. (2007). Design, synthesis, and cytostatic activity of novel cyclic curcumin analogues. Bioorganic & Medicinal Chemistry Letters. 17. 5624-5629.
5. Aggarwal, B.B., Bhatt. I.D.. Ichikawa. H.. Ahn, K.S., Sethi, G,. Sandur, S.K., Sundaram, C., Seeram, N., Shishodia, S. (2007).Curcumin - biological and medicinal properties, in: P.N. Ravindran, K.N. Babu, K. Sivaraman (Eds.), Turmeric the Genus Curcuma, CRC Press. NY, pp. 297-368.
6. Shao, Z.M., Shen, Z.Z., Liu, C.H., Sartippour, M.R., Go, V.L., Heber, D., Nguyen, M. (2002). Curcumin exerts multiple suppressive effects on human breast carcinoma cells. International Journal of Cancer, 98, 234-240.
7. Schindler, R., Mentlein, R. (2006). Flavonoids and vitamin E reduce the release of the angiogenic peptide vascular endothelial growth factor from human tumor cells, Journal of Nutrition, 13δ, 1477-1482.
8. Ramachandran, C., Fonseca, H.B., Jhabvala, F., Escalon, EA., Melnick, S.J. (2002). Curcumin inhibits telomerase activity through human telomerase reverse transcriptase inMCF-7 breast cancer cell line. Cancer Letters, 184, 1-6.
9. Di, GJH., Li, H.C., Shen, Z.Z., Shao, Z.M. (2003). Analysis of antiproliferation of curcumin on human breast cancer cells and its mechanism. Zhonghua Yi Xue Za Zhi, 83, 1764- 1768.
10. Bobrovnikova-Marjon, E.V., Marjon, P.L., Barbash, O., Vander Jagt, D.L., Abcouwer, S.F. (2004). Expression of angiogenic factors vascular endothelial growth factor and interleukin- 8/CXCL8 is highly responsive to ambient glutamine availability: role of nuclear factor-kappaB and activating protein-1. Cancer Research., 64 4858-4869.
11. Aggarwal, B.B., Shishodia, S., Takada, Y., Banerjee, S., Newman, R.A., Bueso-ramos, C.E., Pncc, J.E. (2005). Curcumin suppresses the paclitaxel-induced nuclear factor-kappaB pathway in breast cancer cells and inhibits lung metastasis of human breast cancer in nude mice. Clin. Cancer Research, 11, 7490-7498.
12. Yoon, H., Liu, R.H. (2007). Effect of selected phytochemicals and apple extracts on NF-kappaB activation in human breast cancer MCF-7 cells. Journal of Agricultural and Food Chemistry 55, 3167- 3173.
13. Bachmeier, B.E., Mohrenz, I.Y., Mirisola, V., Schleicher, E., Romeo, F., Hohneke, C., Jochum, M., Nertich, A.G., Pfeffer, U. (2008). Curcumin down-regulates the inflammatory cytokines CXCL1 and -2 in breast cancer cells via NF{kappa} B. Carcinogenesis, 29(4), 779-89.
14. Hammamieh, R., Sumaida, D., Zhang, X., Das, R., Jett, M. (2007). Control of the growth of human breast cancer cells in culture by manipulation of arachidonate metabolism. BMC Cancer, 7, 138.
15. Xia, X., Cheng, G., Pan, Y., Xia, Z.H., Kong, L.D. (2007). Behavioral, neurochemical, neuroendocrine effects of the ethanolic extract from Curcuma longa L.in the Mouse forced swimming test. Journal of Ethnopharmacology, 110, 356- 363.
16. Liao, Z., Cox, J.D., Komati, R. (2007). Radiochemotherapy of esophageal cancer. Journal of Thoracic Oncology. 2. 553-568.
17. Rafiee, P., Ogawa, H.; Heidemann, J., Li, M.S., Aslam, M., Lamirand, T.H., Fisher, P.J., Graewm, S.J_, Dwinell, M.B., Johnson, C.P., Shaker, R., Binion, D.G. (2003). Isolation and characterization of human esophageal microvascular endothelial cells: mechanisms of inflammatory activation. American Journal of Physiology-Gastrointestinal Liver Physiology, 285, 1271- 1292.
18. Goel, A., Kunnumakkara, A.B., Aggarwal, B.B. (2008). Curcumin as Curecumin: from kitchen to clinic, Biochemical Pharmacology, 75(4), 787-809.
19. Tunstall, R.G., Sharma, R.A., Perkins, S., Sale, S., Singh, R., Farmer, P.B., Steward, W.P., Gescher, A.J. (2006). Cyclooxygenase- 2 expression and oxidative DNA adducts in murine intestinal adenomas: modification by dietary curcumin and implications for clinical trials. European Journal of Cancer, 42, 415-421.
20. Vietri, M., Pietrabissa, A., Moaca, F., Spisni, R., Pacifici, G.M. (2003). Curcumin is a potent inhibitor of phenol sulfotransferase (SULT1A1) in human liver and extrahepatic tissues. Xenobiotica. 33, 357-363.
21. Chen, Y.N., Cheng, C.C., Chen, J.C., Tsauer, W., Hsu, S.L. (2003). Norcantharidin-induced apoptosis is via the extracellular signal-regulated kinase and c-Jun-NH2-terminal kinase signaling pathways in human hepatoma HepG2 cells. British Journal of Pharmacology, 140, 461-470.
22. Bae, M.K., Kim, S.H., Jeong, J.W., Lee, Y.M., Kim, H.S., Kim, S.R., Yun, L, Bae, S.K., Kim, K.W. (2006). Curcumin inhibits hypoxia-induced angiogenesis via downregulation of HIF- 1. Oncology Reports, 15,1557-1562.
23. Choi, H., Chun, Y.S., Kim, S_W_, Kim, M.S., Park, J.W. (2006). Curcumin inhibits hypoxiainducible factor-1 by degrading aryl hydrocarbon receptor nuclear translocator: a mechanism of tumor growth inhibition. Molecular Pharmacology, 70, 1664-1671.
24. Aggarwal, B.B., Kumar, A., Bharti, A.C. (2003). Anticancer potential of curcumin: preclinical and clinical studies. Anticancer Research, 23, 363-398.
25. Wang, W., Abbruzzese, J.L., Evans, D.B., Larry, L., Cleary, K.R., Chiso, P.J. (1999). The nuclear Actor-kappa B RelA transcription factor is constitutively activated in human pancreatic adenocarcinoma cells. Clinical Cancer Research, 5, 119-127.
26. Li. L., Aggarwal, B.B., Shishodia, S., Abbruzzese, J., Kurzrock, R. (2004). Nuclear factorkappaB and IkappaB kinase are constitutively active in human pancreatic cells, and their downregulation by curcumin (diferuloylmethane) is associated with the suppression of proliferation and the induction of apoptosis. Cancer, 101, 2351-2362.
27. Khanbolooki, S., Nawrocki, S.T., Arumugam, T., Andtbacka, R_, Pino, M.S., Kurzrock, R., Logsdon, C.D., Abbruzzese, J.L., McConkey DJ. (2006). Nuclear factor-kappaB maintains TRAIL resistance in human pancreatic cancer cells. Molecular Cancer Therapeutics, 5, 2251- 2260.
28. Kamohara, H_, Takahashi, M., Ishiko, T., Ogawa, M., Baba, H. (2007). Induction of interleukin8 (CXCL-8) by tumor necrosis factor-alpha and leukemia inhibitory factor in pancreatic carcinoma cells: impact of CXCL-8 as an autocrine growth factor. International Journal of Oncology, 31, 627-632.
29. Dhillon, N., Aggarwal, B.B., Newman, R.A., Wolff, R.A., Kunnumakkara, A.B., Abbruzzese, J.L., Hong. D.S., Camacho, L.H., Ng, C., Kurzrock, R. (2007). Curcumin and pancreatic cancer: phase II clinical trial experience. Journal of Clinical Oncology, 25,4599.
30. Wei, S.C., Lin, Y.S., Tsao, P.N., Wu-Tsai, J.J., Wu, C.H., Wong, J.M. (2004). Comparison of the anti-proliferation and apoptosisinduction activities of sulindac, celecoxib, curcumin, and nifedipine in mismatch repair-deficient cell lines. Journal of the Formosan Medical Association, 103, 599-606.
31. Arya et al., 20 10 ,A. Arya, A. Chandra, V. Sharma, K. PathakFast dissolving oral films: an innovative drug delivery system and dosage form Int. J. Chem. Tech. Res., 2 (2010), pp. 576-583.
32.Bai2007,G. Bai, P.M. Armenante, R.V. Plank, M. Gentzler, K. Ford. P. HarmonHyd rodynamic investigation of USP dissolution test apparatus IIJ. Pharm. Sci., 96 (2007), pp. 2327-2349

### Figure Legends

Figure 1: Caco-2 Model - Absorption of CAVACURMIN^{®} in Comparison to Leading Commercial Products.
Figure 2: Rodent Model - High Bioavailability of CAVACURMIN^{®} as Measured by Total Curcuminoids.
Figure 3: Clinical Study - Curcumin Blood Concentration

### Object and Brief Description of the Invention

The object of the present invention is to provide a formulation that eliminates the low bioavailability of curcumin, and a dosage form for this formulation.

The present invention is a pharmaceutical composition for use as a food supplement, containing at least one herbal active ingredient, at least one mineral in organic or inorganic form, and at least one excipient. The active ingredient in said composition is obtained from a plant. Said plant is a curcuma longa plant. In the invention, also curcumin and its compounds derived from curcuma longa can be used as active ingredients. Such compounds include the curcumin analogs dimethoxycurcumin, tetrahydrocurcumin and bis-dimethoxycurcumin as well as combinations thereof such as a turmeric (i.e. curcuma longa) extract that contains 95 wt% curcuminoids and where said curcuminoids consist of curcumin, dimethoxycurcumin, and bis-dimethoxycurcumin.

Within the context of the present invention curcumin and its compounds derived from curcuma longa are formulated as a complex with cyclodextrin, i.e.as a gamma-cyclodextrin curcumin complex.

The pharmaceutical composition of the invention for use as a food supplement contains the plant or curcuma longa or curcuma longa-derived curcumin and its compounds in an amount of 1-100%.

The pharmaceutical composition of the invention for use as a food supplement contains selenium as a mineral. The formulation comprises selenium in an amount of 0.1-50 wt%.

The pharmaceutical composition of the invention for use as a food supplement further comprises at least one filler as an excipient. The formulation comprises filler in an amount of 1-50 wt%. The filler in the formulation is selected from at least one or more of "lactose monohydrate, lactose (Lactose) anhydrase, sugar, mannitol, dextrate and derivatives thereof, microcrystalline cellulose, starch, pregelatinized starch, cellulose and fumed silica".

In one embodiment, one of the excipients in the pharmaceutical composition of the invention for use as a food supplement is at least one sweetener. The sweetener is present in the composition in an amount of 1-30 wt%. Said sweetener is selected from at least one of "sodium cyclamate, sodium saccharin, acesulfame K, stevia and aspartame".

In one embodiment, one of the excipients in the pharmaceutical composition of the invention for use as a food supplement is at least one lubricant. The lubricant is present in the composition in an amount of 0.1-3 wt%. Said composition comprises at least one lubricant selected from the group consisting of "magnesium stearate, sodium stearyl fumarate, stearic acid and magnesium stearate".

In one embodiment, the pharmaceutical composition of the invention for use as a food supplement comprises at least one flavouring agent as one of the excipients. The flavouring agent is present in the composition in an amount of 0.1-3 wt%.

The gamma-cyclodextrin curcumin complex mentioned in the following embodiments is the physical blend of gamma-cyclodextrin and turmeric (i.e. curcuma longa) extract in an amount of 95 wt%, where turmeric extract contains 95 wt% curcuminoids and where curcuminoids consist of 65-80 wt% curcumin, 15-20 wt% dimethoxycurcumin, and 15-20 wt% bis-dimethoxycurcumin.

In one embodiment, the pharmaceutical composition of the invention for use as a food supplement consists of 1-96.7 wt% gamma-cyclodextrin curcumin complex, 0.1-50 wt% selenium and/or selenomethionine, 1-50 wt% lactose monohydrate, 1-30 wt% mannitol, 1-30 wt% sodium cyclamate, 0.1-3 wt% magnesium stearate and 0.1-3 wt% flavouring agent.

In another embodiment, the pharmaceutical composition of the invention for use as a food supplement consists of 1-96.7 wt% the gamma-cyclodextrin curcumin complex as well as 0.1-50 wt% selenium and/or selenomethionine, 1-50 wt% maltose, 1-30 wt% sorbitol, 1-30 wt% sodium cyclamate, 0.1-3 wt% magnesium stearate and 0.1-3 wt% flavouring agent.

In another embodiment, the pharmaceutical composition of the invention for use as a food supplement consists of 1-96 wt% gamma-cyclodextrin curcumin complex as well as 0,1-50 wt% selenium and/or selenomethionine, 1-50 wt% dextrate, 1-30 wt% sorbitol, 1-30 wt% acesulfame K, 0.1-3 wt% sodium stearyl fumarate and 0.1-3 wt% flavouring agent.

In another embodiment, the pharmaceutical composition of the invention for use as a food supplement consists of 1-96,7 wt% gamma-cyclodextrin curcumin complex as well as 0,1-50 wt% selenium and/or selenomethionine, 1-50 wt% fumed silica, 1-30 wt% sorbitol, 1-30 wt% acesulfame K, 0.1-3 wt% sodium stearyl fumarate and 0.1-3 wt% flavouring agent.

In another embodiment, the pharmaceutical composition of the invention for use as a food supplement consists of 1-96.7 wt% the gamma-cyclodextrin curcumin complex and 0.1-50 wt% selenium and/or selenomethionine, 1-50 wt% dextrate, 1-30 wt% sorbitol, 1-30 wt% acesulfame K, 0.1-3 wt% stearic acid and 0.1-3 wt% flavouring agent.

In another embodiment, the pharmaceutical composition of the invention for use as a food supplement consists of 1-96.7 wt% the gamma-cyclodextrin curcumin complex and 0.1-50 wt% selenium and/or selenomethionine, 1-50 wt% dextrate, 1-30 wt% maltitol, 1-30 wt% acesulfame K, 0.1-3 wt% stearic acid and 0.1-3 wt% flavouring agent.

In another embodiment, the pharmaceutical composition of the invention for use as a food supplement consists of 1-96.7 wt% the gamma-cyclodextrin curcumin complex and 0.1-50 wt% selenium and/or selenomethionine, 1-50 wt% dextrate, 1-30 wt% mannitol, 1-30 wt% acesulfame K, 0.1-3 wt% magnesium stearate and 0.1-3 wt% flavouring agent.

The dosage form of the pharmaceutical composition of the invention for use as a food supplement is a tablet. Said tablets are in oral dosage form. This is achieved by the appropriate combinations of active ingredients and excipients. Thus, it is easily dispersed and dissolved in the mouth.

The present invention could be summarized by the appended set of claims

### Detailed Description of the Invention

### Selenium

Selenium is a dietary essential mineral. It is found in selenomethionine form in the body. The highest amount of muscle (50%) is present in the kidney, liver, heart and skin. The organs in which it is stored are erythrocytes, spleen, heart, nails, tooth enamel and sperm. It acts as a cofactor of glutathione peroxidase which is one of the main antioxidants in the cell. Its biological effects stem from its selenoprotein content that contains cysteine amino acid. It supports the immune system, thyroid functions and reproductive functions. It inhibits tumour growth. It acts as an antioxidant. It selectively inhibits tumour cells in cancerous tissues. In addition, it is involved in enzymatic activities in the cancer-protective mechanisms. It shows anticarcinogenic effect by inducing apoptosis in cancerous cells. It strengthens the effectiveness of immune cells through specific reactions.

Selenium acts as a glutathione peroxidase enzyme cofactor. Selenium is necessary for the enzyme activity of glutathione peroxidase. Glutathione peroxidase is a selenium-containing antioxidant enzyme. As the level of selenium increases, plasma and liver glutathione peroxidase levels increase as well. It promotes the activity of glutathione, and thus reduces the load of vitamin E in scavenging free radicals.

Inventors have discovered that curcumin is useful as a natural agent, besides its anticancer, antioxidant, antidiabetic, anti-inflammatory, antiallergic, antiartirit, antiallergic, as well as clinical activity, in eliminating, thanks to its chemically non-water soluble but fat-soluble nature as well as associated low absorption, the chemical disadvantages of curcumin that may result from such low absorption properties, and also in enhancing such activities.

The invention relates to a novel formulation and dosage form that eliminates the low bioavailability of curcumin.

The content of the product of the invention:

### • Curcumin

(The active ingredient used in the formulation is, in one embodiment, "curcumin complexed with gamma-cyclodextrin" and commercially available by Wacker under the name Cavacurmin).

Complexed with gamma-cyclodextrin, curcumin, i.e. CAVACURMIN, is the physical blend of gamma-cyclodextrin and 95% turmeric (i.e. Curcuma longa) extract.

Curcuma longa or turmeric extract contains curcuminoids in an amount of 95 wt%. And curcuminoids consist of 65-80 wt% curcumin, 15-20 wt% dimethoxycurcumin and 2-5 wt% bis-demethoxycurcumin.

### • Selenium

and/or selenomethionine form

### Dosage Form of the invention:

### • Oral dissolvable tablet

With the composition formed in our invention, bioavailability was greatly increased. In one embodiment of our invention, "curcumin complexed with gamma-cyclodextrin*" as an active ingredient has 40 times more bioavailability than the current standard curcumin, and this has experimentally been proven by the following studies. In order to exhaustively explain and support the effectiveness of the active ingredient of our invention, we present these studies as follows.

### • CACO-2 cells

In Vitro Bioavailability of curcumin complexed with gamma-cyclodextrin (i.e. Cavacurmin) in Human Caco-2 Model (2011).

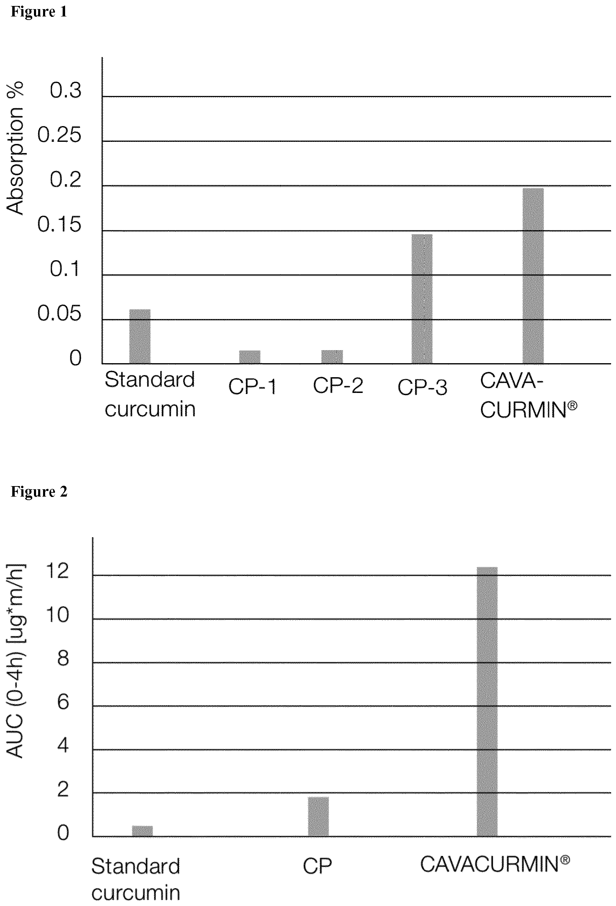

In this study, the bioavailability assay carried out in cell culture between the standard curcumin, two commercially available products (CP) and Cavacurmin is presented. The uptake was up to 10 times higher than for other leading commercial curcumin formulations (CP-1 & CP-2) or curcumin powder itself (see Figure 1).

### • Animal Model

Curcumin Complexed with gamma-cyclodextrin (Cavacurmin) Rodent Model (2011)

In this study, the bioavailability assay carried out in the animal model between the standard curcumin, the commercially available product (CP) and Cavacurmin is presented.

Total concentrations of curcuminoids in the blood plasma (0-4 hours) of Sprague Dawley rats were recorded after one oral gavage (500 mg/kg bw) of three curcumin preparations: standard curcumin extract, brand name curcumin (= CP) and CAVACURMIN^{®}. Plasma was analyzed for free curcumin and curcumin metabolites (curcumin sulfates and curcumin glucuronides) by HPLC (0-4 hours) (AUC values determined).

Animals that received CAVACURMIN^{®} showed a 10 to 20 times higher amount of total curcuminoids in their blood plasma, expressed as the sum of free curcumin and its metabolites, than animals that received a commercial product or pure curcumin powder (see Figure 2).

### • Curcumin complexed with gamma-cyclodextrin (Cavacurmin).

### Human Clinical Study

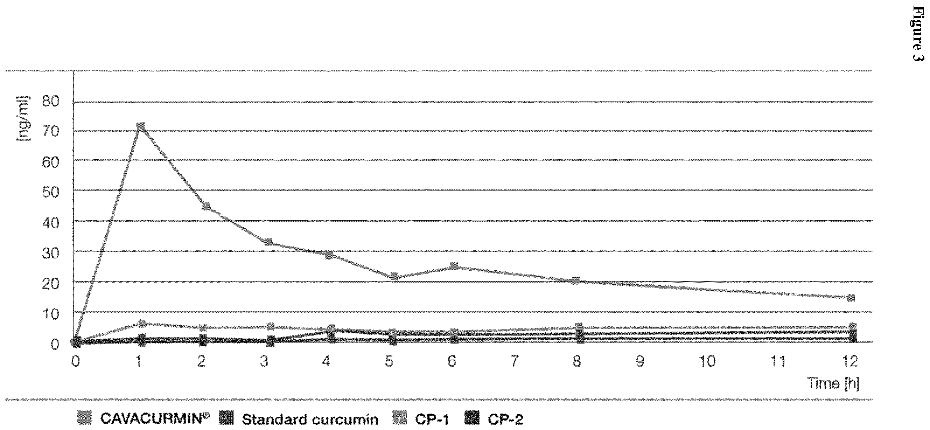

In this study, the bioavailability assay carried out in human between the standard curcumin, two commercially available products (CP) and Cavacurmin is presented. 12 individuals (fasted overnight) were given three different bio-available curcumin preparations and standard curcumin orally - with a one-week washout period in between the four formulations. After product intake, blood was drawn hourly for 12 hours and analyzed (spiked plasma samples). Blood concentration and the relative absorption of curcumin and its derivatives were determined. Curcumin complexed with gamma-cyclodextrin was about 40 times more efficiently absorbed compared to leading commercial curcumin supplement products or curcumin powder itself. The highly superior performance of CAVACURMIN^{®} was demonstrated by the fact that the curcumin uptake was at least 4.6 times higher than the next-best commercial curcumin formulation in this clinical study (see Figure 3).

Similar results have previously been described in the patent applications US2010179103 and WO2018203195 but fail to disclose an oral dispersible tablet as provided by the instant invention. The dosage form of our invention is oral dispersible tablet.

The inventors have developed a dosage form as an oral dispersible tablet for use as a food supplement with the following properties to overcome the disadvantages described in the prior art.
1. It contains curcumin complexed with gamma-cyclodextrin.
2. A formulation structure without unnecessary additives was found to increase bioavailability.
3. The formulation contains selenium.
4. Thanks to the dosage form used, an oral-dispersible dosage form allowing oral absorption has been developed.

With the above formulation, curcumin remains in its natural state and any physical and chemical stability issue is eliminated. Such formulation will ensure that the aforementioned clinical advantages are met by the patient.

Plenty of excess excipients have been excluded in the formula also in order to eliminate the acute toxicities of ionic or non-ionic surfactants which may cause side effects in the formulation of the invention and cause the curcumin to dissolve. Said ionic or non-ionic surfactants are currently used commercially in many preparations containing curcumin. Furthermore, thanks to both its capability to enhance bioavailability and its antioxidant effect, the presence of selenium has created a synergistic effect for curcumin.

In addition, various formulation studies have been carried out in the present invention to suppress the taste of curcumin and selenium, and a formulation offering the most optimum physical properties has been obtained. While conducting this study, researchers have employed volumizing sweeteners, natural sweeteners and tablet lubricants as well as flavouring agents in the formulation for optimum effect.

### Exemplary Formulation-1

| Agent | Unit Formula wt% | Function |
|---|---|---|
| * as gamma-cyclodextrin curcumin complex | 1-100 | Active Ingredient |
| Selenium | 0.1-50 | Active Ingredient |
| Lactose Monohydrate | 1-50 | Filler |
| Mannitol | 1-30 | Filler |
| Sodium Cyclamate | 1-30 | Sweetener |
| Magnesium Stearate | 0.1-3 | Lubricant |
| Aroma | 0.1-3 | Flavouring Agent |

In exemplary formulation-1, gamma-cyclodextrin curcumin in complex form and blended selenium, lactose monohydrate, mannitol, sodium cyclamate and magnesium stearate are dosed into tablets.

### Exemplary Formulation-2

| Agent | Unit Formula wt% | Function |
|---|---|---|
| * as gamma-cyclodextrin curcumin complex | 1-100 | Active Ingredient |
| Selenium | 0.1-50 | Active Ingredient |
| Maltose | 1-50 | Filler |
| Sorbitol | 1-30 | Filler |
| Sodium Cyclamate | 1-30 | Sweetener |
| Magnesium Stearate | 0.1-3 | Lubricant |
| Aroma | 0.1-3 | Flavouring Agent |

In exemplary formulation-2, gamma-cyclodextrin curcumin in complex form and blended selenium, maltose, sorbitol, sodium cyclamate and magnesium stearate are dosed into tablets.

### Exemplary Formulation-3

| Agent | Unit Formula wt% | Function |
|---|---|---|
| * as gamma-cyclodextrin curcumin complex | 1-100 | Active Ingredient |
| Selenium | 0.1-50 | Active Ingredient |
| Dextrate | 1-50 | Filler |
| Sorbitol | 1-30 | Filler |
| Acesulfame K | 1-30 | Sweetener |
| Sodium stearyl fumarate | 0.1-3 | Lubricant |
| Aroma | 0.1-3 | Flavouring Agent |

In exemplary formulation-3, gamma-cyclodextrin curcumin in complex form and blended selenium, dextrate, sorbitol, acesulfame K, sodium stearyl fumarate are dosed into tablets.

### Exemplary Formulation-4

| Agent | Unit Formula wt% | Function |
|---|---|---|
| * as gamma-cyclodextrin curcumin complex | 1-100 | Active Ingredient |
| Selenium | 0.1-50 | Active Ingredient |
| Fumed Silica | 1-50 | Filler |
| Sorbitol | 1-30 | Filler |
| Acesulfame K | 1-30 | Sweetener |

| Agent | Unit Formula wt% | Function |
|---|---|---|
| Sodium stearyl fumarate | 0.1-3 | Lubricant |
| Aroma | 0.1-3 | Flavouring Agent |

In exemplary formulation-4, gamma-cyclodextrin curcumin in complex form and blended selenium, fumed silica, sorbitol, acesulfame K and sodium stearyl fumarate are dosed into tablets.

### Exemplary Formulation-5

| Agent | Unit Formula wt% | Function |
|---|---|---|
| * as gamma-cyclodextrin curcumin complex | 1-100 | Active Ingredient |
| Selenium | 0.1-50 | Active Ingredient |
| Dextrate | 1-50 | Filler |
| Sorbitol | 1-30 | Filler |
| Acesulfame K | 1-30 | Sweetener |
| Stearic Acid | 0.1-3 | Lubricant |
| Aroma | 0.1-3 | Flavouring Agent |

In exemplary formulation-5, gamma-cyclodextrin curcumin in complex form and blended selenium, dextrate, sorbitol, acesulfame K and stearic acid are dosed into tablets.

### Exemplary Formulation-6

| Agent | Unit Formula wt% | Function |
|---|---|---|
| * as gamma-cyclodextrin curcumin complex | 1-100 | Active Ingredient |
| Selenium | 0.1-50 | Active Ingredient |
| Dextrate | 1-50 | Filler |
| Maltitol | 1-30 | Filler |
| Acesulfame K | 1-30 | Sweetener |
| Stearic Acid | 0.1-3 | Lubricant |
| Aroma | 0.1-3 | Flavouring Agent |

In exemplary formulation-6, gamma-cyclodextrin curcumin in complex form and blended selenium, dextrate, maltitol, acesulfame K and stearic acid are dosed into tablets.

### Exemplary Formulation-7

| Agent | Unit Formula wt% | Function |
|---|---|---|
| * as gamma-cyclodextrin curcumin complex | 1-100 | Active Ingredient |
| Selenium | 0.1-50 | Active Ingredient |
| Dextrate | 1-50 | Filler |
| Mannitol | 1-30 | Filler |
| Acesulfame K | 1-30 | Sweetener |
| Magnesium Stearate | 0.1-3 | Lubricant |
| Aroma | 0.1-3 | Flavouring Agent |

In exemplary formulation-7, gamma-cyclodextrin curcumin in complex form and blended selenium, dextrate, mannitol, acesulfame K and magnesium stearate are dosed into tablets.

The summary table and the results of the abovementioned assays are presented below.

| Agent | Smell | Taste | Harmony | Oral Absorption |
|---|---|---|---|---|
| Formulation 1 | * | * | * | *** |
| Formulation 2 | ** | ** | ** | *** |
| Formulation 3 | ** | *** | *** | *** |
| Formulation 4 | *** | **** | **** | **** |
| Formulation 5 | *** | **** | **** | **** |
| Formulation 6 | **** | **** | **** | **** |
| Formulation 7 | ***** | ***** | ***** | ***** |

| | | | | |
|---|---|---|---|---|
| *****: Very good ****: Good ***: Intermediate **: Low *: Poor | | | | |

## Claims

1. Pharmaceutical composition suitable for use as a food supplement containing
- gamma cyclodextrin curcumin complex;
- selenium and/or selenomethionine in organic or inorganic form as a mineral;
- one or more filler selected from lactose monohydrate, lactose anhydrase, sugar, mannitol, dextrate and derivatives thereof, microcrystalline cellulose, starch, pregelatinized starch, cellulose and fumed silica;
- a lubricant selected from magnesium stearate, stearic acid and sodium sterile fumarate;
- a sweetener; and
- a flavouring agent.

2. Pharmaceutical composition according to claim 1, wherein it contains gamma cyclodextrin curcumin complex in an amount of 1 to 100 wt%.

3. Pharmaceutical composition according to claim 1, wherein it contains 0.1 to 50 wt% selenium in organic or inorganic form.

4. Pharmaceutical composition according to Claim 1, wherein the one or more filler is selected from fumed silica, sorbitol, dextrose, mannitol and maltitol.

5. Pharmaceutical composition according to any one of the preceding claims, wherein it comprises 1 to 50 wt% filler.

6. Pharmaceutical composition according to Claim 1, wherein it contains at least one of sodium cyclamate, sodium saccharin, acesulfame K, stevia and aspartame as the sweetener.

7. Pharmaceutical composition according to claims 1 or 6, wherein it comprises 1 to 30 wt% sweetener.

8. Pharmaceutical composition according to claim 1, wherein it comprises 0.1 to 3 wt% lubricant.

9. Pharmaceutical composition according to claim 1, wherein it comprises 0.1 to 3 wt% flavouring agent.

10. Pharmaceutical composition of any of the claims 1 to 9 as a food supplement, said pharmaceutical composition comprising 1-96.7 wt% gamma cyclodextrin curcumin complex and 0.1-50 wt% selenium and/or selenomethionine, 1-50 wt% fumed silica, 1-30 wt% sorbitol, 1-30 wt% acesulfame K, 0.1-3 wt% sodium stearyl fumarate and 0.1-3 wt% flavouring agent, wherein, in this composition: gamma-cyclodextrin curcumin complex is the physical blend of gamma-cyclodextrin and 95 wt% turmeric (i.e. curcuma longa) extract, wherein turmeric extract in this physical blend contains 95 wt% curcuminoids and wherein curcuminoids consist of 65-80 wt% curcumin, 15-20 wt% dimethoxycurcumin, and 15-20 wt% bis-dimethoxycurcumin.

11. Pharmaceutical composition of any of the claims 1 to 9 as a food supplement, said pharmaceutical composition comprising 1-96.7 wt% gamma cyclodextrin curcumin complex and 0.1-50 wt% selenium and/or selenomethionine, 1-50 wt% dextrate, 1-30 wt% sorbitol, 1-30 wt% acesulfame K, 0.1-3 wt% stearic acid and 0.1-3 wt% flavouring agent, wherein, in this composition: gamma-cyclodextrin curcumin complex is the physical blend of gamma-cyclodextrin and 95 wt% turmeric (i.e. curcuma longa) extract, wherein turmeric extract in this physical blend contains 95 wt% curcuminoids and wherein curcuminoids consist of 65-80 wt% curcumin, 15-20 wt% dimethoxycurcumin, and 15-20 wt% bis-dimethoxycurcumin.

12. Pharmaceutical composition of any of the claims 1 to 9 as a food supplement, said pharmaceutical composition comprising 1-96.7 wt% gamma cyclodextrin curcumin complex and 0.1-50 wt% selenium and/or selenomethionine, 1-50 wt% dextrate, 1-30 wt% maltitol, 1-30 wt% acesulfame K, 0.1-3 wt% stearic acid and 0.1-3 wt% flavouring agent, wherein, in this composition: gamma-cyclodextrin curcumin complex is the physical blend of gamma-cyclodextrin and 95 wt% turmeric (i.e. curcuma longa) extract, wherein turmeric extract in this physical blend contains 95 wt% curcuminoids and wherein curcuminoids consist of 65-80 wt% curcumin, 15-20 wt% dimethoxycurcumin, and 15-20 wt% bis-dimethoxycurcumin.

13. Pharmaceutical composition of any of the claims 1 to 9 as a food supplement, said pharmaceutical composition comprising 1-96.7 wt% gamma cyclodextrin curcumin complex and 0.1-50 wt% selenium and/or selenomethionine, 1-50 wt% dextrate, 1-30 wt% mannitol, 1-30 wt% acesulfame K, 0.1-3 wt% magnesium stearate and 0.1-3 wt% flavouring agent, wherein, in this composition: gamma-cyclodextrin curcumin complex is the physical blend of gamma-cyclodextrin and 95 wt% turmeric (i.e. curcuma longa) extract, wherein turmeric extract in this physical blend contains 95 wt% curcuminoids and wherein curcuminoids consist of 65-80 wt% curcumin, 15-20 wt% dimethoxycurcumin, and 15-20 wt% bis-dimethoxycurcumin.

14. Tablet comprising the pharmaceutical composition of any of the preceding claims.

15. Tablet according to Claim 14, wherein the active ingredient and excipients together with their combinations mentioned in the preceding claims are in oral-dispersible dosage form.

## Patentansprüche

1. Pharmazeutische, zur Verwendung als Nahrungsergänzungsmittel geeignete Zusammensetzung, welche folgende Inhaltsstoffe enthält:
Gamma-Cyclodextrin-Curcumin-Komplex;
Selen und/oder Selenomethionin in organischer oder anorganischer Form als Mineralstoff;
einen oder mehrere Füllstoffe, ausgewählt aus der Gruppe bestehend aus Laktosemonohydrat, Laktoseanhydrase, Zucker, Mannitol, Dextrat und Derivaten davon, mikrokristalliner Cellulose, Stärke, vorgelatinierter Stärke, Cellulose und pyrogener Kieselsäure;
ein Gleitmittel, ausgewählt aus der Gruppe bestehend aus Magnesiumstearat, Stearinsäure und Natriumsterilfumarat;
ein Süßungsmittel; und
einen Aromastoff.

2. Pharmazeutische Zusammensetzung nach Anspruch 1, **dadurch gekennzeichnet, dass** diese einen Gamma-Cyclodextrin-Curcumin-Komplex in einer Menge von 1 bis 100 Gew.-% enthält.

3. Pharmazeutische Zusammensetzung nach Anspruch 1, **dadurch gekennzeichnet, dass** diese 0,1 bis 50 Gew.-% Selen in organischer oder anorganischer Form enthält.

4. Pharmazeutische Zusammensetzung nach Anspruch 1, **dadurch gekennzeichnet, dass** der eine oder die mehreren Füllstoffe ausgewählt sind aus der Gruppe bestehend aus pyrogener Kieselsäure, Sorbit, Dextrose, Mannit und Maltit.

5. Pharmazeutische Zusammensetzung nach einem der vorherigen Ansprüche, **dadurch gekennzeichnet, dass** diese 1 bis 50 Gew.-% Füllstoff enthält.

6. Pharmazeutische Zusammensetzung nach Anspruch 1, **dadurch gekennzeichnet, dass** diese als Süßungsmittel Natriumcyclamat und/oder Natriumsaccharin und/oder Acesulfam K und/oder Stevia und/oder Aspartam enthält.

7. Pharmazeutische Zusammensetzung nach Anspruch 1 oder 6, **dadurch gekennzeichnet, dass** diese 1 bis 30 Gew.-% Süßungsmittel enthält.

8. Pharmazeutische Zusammensetzung nach Anspruch 1, **dadurch gekennzeichnet, dass** diese 0,1 bis 3 Gew.-% Gleitmittel enthält.

9. Pharmazeutische Zusammensetzung nach Anspruch 1, **dadurch gekennzeichnet, dass** diese 0,1 bis 3 Gew.-% Aromastoffe enthält.

10. Pharmazeutische Zusammensetzung nach einem der Ansprüche 1 bis 9 als Nahrungsergänzungsmittel,
**dadurch gekennzeichnet, dass** diese 1 bis 96,7 Gew.-% Gamma-Cyclodextrin-Curcumin-Komplex und 0,1 bis 50 Gew.-% Selen und/oder Selenomethionin, 1 bis 50 Gew.-% pyrogene Kieselsäure, 1 bis 30 Gew.-% Sorbit, 1 bis 30 Gew.-% Acesulfam K, 0,1 bis 3 Gew.-% Natriumstearylfumarat und 0,1 bis 3 Gew.-% Aromastoff aufweist, wobei, in dieser Zusammensetzung, Gamma-Cyclodextrin-Curcumin-Komplex die physikalische Mischung aus Gamma-Cyclodextrin und 95 Gew.-% Kurkuma (d.h. Curcuma longa)-Extrakt darstellt, das Kurkuma-Extrakt in dieser physikalischen Mischung 95 Gew.-% Curcuminoide enthält, und wobei die Curcuminoide aus 65 bis 80 Gew.-% Curcumin, 15 bis 20 Gew.-% Dimethoxycurcumin und 15 bis 20 Gew.-% Bis-Dimethoxycurcumin bestehen.

11. Pharmazeutische Zusammensetzung nach einem der Ansprüche 1 bis 9 als Nahrungsergänzungsmittel,
**dadurch gekennzeichnet, dass** diese 1 bis 96,7 Gew.-% Gamma-Cyclodextrin-Curcumin-Komplex und 0,1 bis 50 Gew.-% Selen und/oder Selenomethionin, 1 bis 50 Gew.-% Dextrat, 1 bis 30 Gew.-% Sorbit, 1 bis 30 Gew.-% Acesulfam K, 0,1 bis 3 Gew.-% Stearinsäure und 0,1 bis 3 Gew.-% Aromastoff aufweist, wobei, in dieser Zusammensetzung, Gamma-Cyclodextrin-Curcumin-Komplex die physikalische Mischung aus Gamma-Cyclodextrin und 95 Gew.-% Kurkuma (d.h. Curcuma longa)-Extrakt darstellt, das Kurkuma-Extrakt in dieser physikalischen Mischung 95 Gew.-% Curcuminoide enthält, und wobei die Curcuminoide aus 65 bis 80 Gew.-% Curcumin, 15 bis 20 Gew.-% Dimethoxycurcumin und 15 bis 20 Gew.-% Bis-Dimethoxycurcumin bestehen.

12. Pharmazeutische Zusammensetzung nach einem der Ansprüche 1 bis 9 als Nahrungsergänzungsmittel,
**dadurch gekennzeichnet, dass** diese 1 bis 96,7 Gew.-% Gamma-Cyclodextrin-Curcumin-Komplex und 0,1 bis 50 Gew.-% Selen und/oder Selenomethionin, 1 bis 50 Gew.-% Dextrat, 1 bis 30 Gew.-% Maltit, 1 bis 30 Gew.-% Acesulfam K, 0,1 bis 3 Gew.-% Stearinsäure und 0,1 bis 3 Gew.-% Aromastoff aufweist, wobei, in dieser Zusammensetzung, Gamma-Cyclodextrin-Curcumin-Komplex die physikalische Mischung aus Gamma-Cyclodextrin und 95 Gew.-% Kurkuma (d. h. Curcuma longa) Extrakt darstellt, das Kurkumaextrakt in dieser physikalischen Mischung 95 Gew.-% Curcuminoide enthält, und wobei die Curcuminoide aus 65 bis 80 Gew.-% Curcumin, 15 bis 20 Gew.-% Dimethoxycurcumin und 15 bis 20 Gew.-% Bis-Dimethoxycurcumin bestehen.

13. Pharmazeutische Zusammensetzung nach einem der Ansprüche 1 bis 9 als Nahrungsergänzungsmittel,
**dadurch gekennzeichnet, dass** diese 1 bis 96,7 Gew.-% Gamma-Cyclodextrin-Curcumin-Komplex und 0,1 bis 50 Gew.-% Selen und/oder Selenomethionin, 1 bis 50 Gew.-% Dextrat, 1 bis 30 Gew.-% Mannit, 1 bis 30 Gew.-% Acesulfam K, 0,1-3 Gew.-% Magnesiumstearat und 0,1 bis 3 Gew.-% Aromastoffe aufweist, wobei, in dieser Zusammensetzung, der Gamma-Cyclodextrin-Curcumin-Komplex die physikalische Mischung aus Gamma-Cyclodextrin und 95 Gew.-% Kurkuma (d.h. Curcuma longa)-Extrakt ist, das Kurkumaextrakt in dieser physikalischen Mischung 95 Gew.-% Curcuminoide enthält, und wobei die Curcuminoide aus 65 bis 80 Gew.-% Curcumin, 15 bis 20 Gew.-% Dimethoxycurcumin und 15 bis 20 Gew.-% Bis-Dimethoxycurcumin bestehen.

14. Tablette, aufweisend die pharmazeutische Zusammensetzung nach einem der vorherigen Ansprüche.

15. Tablette nach Anspruch 14, **dadurch gekennzeichnet, dass** der Wirkstoff und die Hilfsstoffe zusammen mit ihren in den vorherigen Ansprüchen genannten Kombinationen in oral einzunehmender dispergierbarer Darreichungsform vorliegen.

## Revendications

1. Composition pharmaceutique adaptée à une utilisation comme complément alimentaire contenant
- le complexe de curcumine gamma-cyclodextrine ;
- le sélénium et/ou la sélénométhionine sous forme organique ou inorganique en tant que minéral,
- une ou plusieurs charges choisies parmi le lactose monohydraté, le lactose anhydre, le sucre, le mannitol, le dextrate et des dérivés de celui-ci, la cellulose microcristalline, l'amidon, l'amidon prégélatinisé, la cellulose et la silice fumée ;
- un lubrifiant choisi parmi le stéarate de magnésium, l'acide stéarique et le stéarylfumarate de sodium ;
- un édulcorant ; et
- un agent aromatisant.

2. Composition pharmaceutique selon la revendication 1, dans laquelle elle contient 1 à 100 % en poids de complexe de curcumine gamma-cyclodextrine.

3. Composition pharmaceutique selon la revendication 1, dans laquelle elle contient 0,1 à 50 % en poids de sélénium sous forme organique ou inorganique.

4. Composition pharmaceutique selon la revendication 1, dans laquelle les une ou plusieurs charges sont choisies parmi la silice fumée, le sorbitol, le dextrose, le mannitol et le maltitol.

5. Composition pharmaceutique selon l'une quelconque des revendications précédentes, dans laquelle elle comprend 1 à 50 % en poids de charge.

6. Composition pharmaceutique selon la revendication 1, dans laquelle elle contient au moins l'un(e) parmi le cyclamate de sodium, la saccharine sodique, l'acésulfame K, la stévia et l'aspartame comme édulcorant.

7. Composition pharmaceutique selon la revendication 1 ou 6, dans laquelle elle comprend 1 à 30 % en poids d'édulcorant.

8. Composition pharmaceutique selon la revendication 1, dans laquelle elle comprend 0,1 à 3 % en poids de lubrifiant.

9. Composition pharmaceutique selon la revendication 1, dans laquelle elle comprend 0,1 à 3 % en poids d'agent aromatisant.

10. Composition pharmaceutique selon l'une quelconque des revendications 1 à 9 en tant que complément alimentaire, ladite composition pharmaceutique comprenant 1 à 96,7 % en poids de complexe de curcumine gamma-cyclodextrine et 0,1 à 50 % en poids de sélénium et/ou de sélénométhionine, 1 à 50 % en poids de silice fumée, 1 à 30 % en poids de sorbitol, 1 à 30 % en poids d'acésulfame K, 0,1 à 3 % en poids de stéarylfumarate de sodium et 0,1 à 3 % en poids d'agent aromatisant, dans laquelle, dans cette composition : le complexe de curcumine gamma-cyclodextrine est le mélange physique de gamma-cyclodextrine et 95 % en poids d'extrait de curcuma (c'est-à-dire curcuma longa), dans laquelle l'extrait de curcuma dans ce mélange physique contient 95 % en poids de curcuminoïdes et dans laquelle les curcuminoïdes sont constitués par 65 à 80 % en poids de curcumine, 15 à 20 % en poids de diméthoxycurcumine et 15 à 20 % en poids de bis-diméthoxycurcumine.

11. Composition pharmaceutique selon l'une quelconque des revendications 1 à 9 en tant que complément alimentaire, ladite composition pharmaceutique comprenant 1 à 96,7 % en poids de complexe de curcumine gamma-cyclodextrine et 0,1 à 50 % en poids de sélénium et/ou de sélénométhionine, 1 à 50 % en poids de dextrate, 1 à 30 % en poids de sorbitol, 1 à 30 % en poids d'acésulfame K, 0,1 à 3 % en poids d'acide stéarique et 0,1 à 3 % en poids d'agent aromatisant, dans laquelle, dans cette composition : le complexe de curcumine gamma-cyclodextrine est le mélange physique de gamma-cyclodextrine et 95 % en poids d'extrait de curcuma (c'est-à-dire curcuma longa), dans laquelle l'extrait de curcuma dans ce mélange physique contient 95 % en poids de curcuminoïdes et dans laquelle les curcuminoïdes sont constitués par 65 à 80 % en poids de curcumine, 15 à 20 % en poids de diméthoxycurcumine et 15 à 20 % en poids de bis-diméthoxycurcumine.

12. Composition pharmaceutique selon l'une quelconque des revendications 1 à 9 en tant que complément alimentaire, ladite composition pharmaceutique comprenant 1 à 96,7 % en poids de complexe de curcumine gamma-cyclodextrine et 0,1 à 50 % en poids de sélénium et/ou de sélénométhionine, 1 à 50 % en poids de dextrate, 1 à 30 % en poids de maltitol, 1 à 30 % en poids d'acésulfame K, 0,1 à 3 % en poids d'acide stéarique et 0,1 à 3 % en poids d'agent aromatisant, dans laquelle, dans cette composition : le complexe de curcumine gamma-cyclodextrine est le mélange physique de gamma-cyclodextrine et de 95 % en poids d'extrait de curcuma (c'est-à-dire curcuma longa), dans laquelle l'extrait de curcuma dans ce mélange physique contient 95 % en poids de curcuminoïdes et dans laquelle les curcuminoïdes sont constitués par 65 à 80 % en poids de curcumine, 15 à 20 % en poids de diméthoxycurcumine et 15 à 20 % en poids de bis-diméthoxycurcumine.

13. Composition pharmaceutique selon l'une quelconque des revendications 1 à 9 en tant que complément alimentaire, ladite composition pharmaceutique comprenant 1 à 96,7 % en poids de complexe de curcumine gamma-cyclodextrine et 0,1 à 50 % en poids de sélénium et/ou de sélénométhionine, 1 à 50 % en poids de dextrate, 1 à 30 % en poids de mannitol, 1 à 30 % en poids d'acésulfame K, 0,1 à 3 % en poids de stéarate de magnésium et 0,1 à 3 % en poids d'agent aromatisant, dans laquelle, dans cette composition : le complexe de curcumine gamma-cyclodextrine est le mélange physique de gamma-cyclodextrine et de 95 % en poids d'extrait de curcuma (c'est-à-dire curcuma longa), dans laquelle l'extrait de curcuma dans ce mélange physique contient 95 % en poids de curcuminoïdes et dans laquelle les curcuminoïdes sont constitués par 65 à 80 % en poids de curcumine, 15 à 20 % en poids de diméthoxycurcumine et 15 à 20 % en poids de bis-diméthoxycurcumine.

14. Comprimé comprenant la composition pharmaceutique selon l'une quelconque des revendications précédentes.

15. Comprimé selon la revendication 14, dans lequel l'ingrédient actif et les excipients ainsi que leurs combinaisons mentionnées dans les revendications précédentes sont sous une forme posologique dispersible administrée par voie orale.
